Europäisches Patentamt

(19) European Patent Office

Office européen des brevets

(11) Publication number : **0 203 768 B2**

## (12) NEW EUROPEAN PATENT SPECIFICATION

(45) Date of publication of the new patent specification : **26.05.93 Bulletin 93/21**

(51) Int. Cl.⁵ : **A61K 9/46, A61K 31/165**

(21) Application number : **86303814.7**

(22) Date of filing : **20.05.86**

(54) **A therapeutic effervescent composition and a method of preparing the same.**

(30) Priority : **31.05.85 US 740144**
**30.08.85 US 775931**
**15.01.86 US 819093**

(43) Date of publication of application :
**03.12.86 Bulletin 86/49**

(45) Publication of the grant of the patent :
**01.08.90 Bulletin 90/31**

(45) Mention of the opposition decision :
**26.05.93 Bulletin 93/21**

(84) Designated Contracting States :
**AT BE CH DE FR GB IT LI LU NL SE**

(56) References cited :
**AT-B- 329 763**
**DE-A- 2 842 822**
**GB-A- 1 328 591**

(56) References cited :
**US-A- 3 773 922**
**US-A- 3 882 228**
**US-A- 4 093 710**
**Exp. Congr. Technol. Pharm. 3rd, 1983,38-48,**
**Die Tablette, Bd.7, 1977,48**
**Acta Pharm Technol, 30(3), 1984, 239-242**
**The theorie and practice of Ind.**
**Pharm,1976,Lea & Febinger, Philadelphia,**
**482-483**

(73) Proprietor : **WARNER-LAMBERT COMPANY**
**201 Tabor Road**
**Morris Plains New Jersey 07950 (US)**

(72) Inventor : **Schobel, Alexander M.**
**610 Somerset Street**
**North Plainfield, NJ 07060 (US)**

(74) Representative : **Jones, Michael Raymond et al**
**HASELTINE LAKE & CO. Hazlitt House 28**
**Southampton Buildings Chancery Lane**
**London WC2A 1AT (GB)**

EP 0 203 768 B2

**Description**

This invention relates to effervescent compositions in the form of tablets or granules which dissolve rapidly in water to yield an effervescent solution containing a completely dissolved therapeutic agent. The invention also relates to the method by which the therapeutic effervescent compositions may be prepared.

More particularly, the invention relates to a novel, analgesic containing effervescent tablet which completely dissolves in cold water in about 1 minute without stirring to yield a palatable effervescent solution of analgesic.

The use of effervescent compositions as a means of administering solubilized therapeutic agents is well known. Dosage forms containing solubilized therapeutic agents have the advantage of being faster acting because they are ready for absorption. When an undissolved therapeutic agent is ingested, it must be dispersed and then solubilized before absorption can take place. All references to drug or therapeutic agent solubility herein refer to solubility in aqueous media.

Effervescent compositions for soluble therapeutic agents are prepared containing the drug to be administered admixed with an effervescent system. Such systems usually comprise an acidifying agent and a carbonate containing compound. When introduced to water, the carbonate containing compound reacts with the acidifying agent to produce a rapid evolution of carbon dioxide gas. This rapid evolution of gas stirs the solution dispersing the therapeutic agent. The stirring is intended to solubilize the therapeutic agent.

Merely placing a therapeutic agent in an effervescent composition does not, however, assure solubilization. The therapeutic agent may be only slightly water soluble and/or may not be wetted by the effervescing solution. A slightly water soluble compound is soluble in from 100 to 1000 parts of solvent per 1 part compound. Achieving complete solubilization of a therapeutic agent is essential to achieving rapid therapeutic relief and avoiding the unpleasant mouthfeel associated with ingesting powder-like suspensions of medicinals. Much effort has been directed toward solubilizing therapeutic agents using effervescent formulations.

U.S. Patent 3,882,228 to Boncey, et al., discloses preparing wettable aspirin particles. The aspirin particles are coated in a spray drying process with a mixture of water soluble coating material, a wetting agent/or a water soluble film forming agent. The coated aspirin particles are then incorporated into effervescent tablets.

British Patent 1,328,591 to Bru discloses a soluble acetaminophen effervescent composition. To achieve solubilization of the acetaminophen, Bru mixes the acetaminophen and the carbonate part of the effervescent carbonate and acid couple into a paste with the aid of an appropriate solvent, such as ethanol, then dries and powders the paste. The acid part of the effervescent couple is then mixed with the powdered paste. Vitamin C is then blended with the dried acetaminophen, carbonate and acid containing particles. The resultant blend is then formed into effervescent tablets.

Effervescent products prepared by these techniques are expensive and difficult to process.

It has unexpectedly been discovered that an effervescent composition can be prepared which may contain up to 30% by weight of a therapeutic agent which when placed in cold water will completely dissolve in less than 1 minute leaving a clear effervescent solution.

In particular, it has been found that a therapeutic effervescent composition which dissolves rapidly in cold water to form a clear solution is produced from an admixture of

(1) a preblended mixture of

(A) a granulated therapeutic agent having a particle size of 100 to 600 $\mu$m, and

(B) a component of an effervescent system having a particle size of 50 to 600 $\mu$m, and

(2) an effervescent system.

While the invention is not limited to theoretical considerations, it is believed that rapid and complete solubilization of the therapeutic agent is achieved through the interaction of several factors.

It is believed that the component of the effervescent system having a particle size of 50 to 600 microns because of its controlled particle size forms a uniform mixture with the granulated therapeutic agent which has about the same particle size. It is further believed that these particulate materials adhere to each other after mixing with the remaining components of the effervescent composition and that this property results in the formation of an extremely uniform effervescent composition which rapidly disintegrates in cold water dispersing the granulated therapeutic agent. It is believed that granulating prevents segregation of the therapeutic agent during mixing to form the effervescent composition and prevents agglomeration in solution after its release from the composition.

The particles of therapeutic agent are of a specific bulk density to optimize the rate of solubilization. Particles of very low bulk density do not wet well when released from an effervescent composition and form a floating film on the solution surface. Particles of a high bulk density fall to the bottom of the solution and dissolve slowly, apparently due to decreased stirring at the bottom of the solution and decreased surface area of the particles.

It is believed that all of the above factors work together to form a rapid dissolving effervescent composition of a therapeutic agent.

Accordingly, a rapid dissolving, effervescent composition containing a therapeutic agent which forms a clear solution in cold water comprises: a preblended mixture present in an amount from 7 to 57.5% by weight of

(A) a granulated therapeutic agent having a particle size of 100 to 600 microns of
(1) a therapeutic agent in an amount from 2 to 27% by weight, and
(2) a granulating agent present in an amount from 0.03 to 2.5% by weight, and
(B) a component of an effervescent system having a particle size from 50 to 600 μm in an amount from 5 to 30% by weight, and

an effervescent system in an amount from 42.5 to 90% by weight has been discovered.

In the instant invention, the therapeutic agent can be any soluble or slightly soluble therapeutic agent or nutrient suitable for oral administration in an aqueous solution.

Suitable therapeutic agents that may be employed in the instant composition may vary widely and generally represent any stable therapeutic agent and combination of therapeutic agents. Illustrative categories and specific examples include:

(a) Antitussives, such as dextromethorphan, dextromethorphan hydrobromide, noscapine, carbetapentane citrate, and chlophedianol hydrochloride;
(b) Antihistamines, such as chlorpheniramine maleate, phenindamine tartrate, pyrilamine maleate, doxylamine succinate, and phenyltoloxamine citrate;
(c) Decongestants, such as phenylephrine hydrochloride, phenylpropanolamine hydrochloride, pseudoephedrine hydrochloride, ephedrine;
(d) Various alkaloids, such as codeine phosphate, codeine sulfate and morphine;
(e) Mineral supplements such as potassium chloride and calcium carbonates, magnesium oxide and other alkali metal and alkaline earth metal salts;
(f) Laxatives, vitamins and antacids;
(g) Ion exchange resins such as cholestyramine;
(h) Anti-cholesterolemic and anti-lipid agents;
(j) Antipyretics and analgesics such as acetaminophen, aspirin and ibuprofen;
(k) Appetite suppressants such as phenylpropanolamine hydrochloride or caffeine;
(l) Expectorants such as guaifenesin;
(m) Anti-inflammatory agents such as isoxicam, and meclophenamic acid; and
(n) Antibiotics such as neomycin, tetracycline, and the polymixins.

Additional useful therapeutic agents include coronary dilators, cerebral dilators, peripheral vasodilators, anti-infectives, psychotropics, antimanics, stimulants, gastro-intestinal sedatives, antidiarrheal preparations, anti-anginal drugs, vasodialators, anti-hypertensive drugs, vasoconstrictors and migraine treatments, tranquilizers, antipsychotics, antitumor drugs, anticoagulants and antithrombotic drugs, hypnotics, sedatives, antiemetics, anti-nauseants, anticonvulsants, neuromuscular drugs, hyper- and hypoglycaemic agents, thyroid and antithyroid preparations, diuretics, antispasmodics, uterine relaxants, mineral and nutritional additives, antiobesity drugs, anabolic drugs, erythropoietic drugs, antiasthmatics, expectorants, cough suppressants, mucolytics, anti-uricemic drugs, and the like.

Mixtures of the therapeutic agents may also be used.

The therapeutic agent is present in an therapeutically effective amount from 2 to 27% by weight of the effervescent composition. In a preferred embodiment, the therapeutic agent is an analgesic selected from the group consisting of acetaminophen, aspirin, ibuprofen, and the like, and mixtures thereof. In a more preferred embodiment, the therapeutic agent is acetaminophen having a bulk density from 0.2 to 0.6 g/ml. The usual dose of acetaminophen is 325 to 1000 mg every four hours. A bulk density less than 0.2 g/ml leaves a powdery mass floating on the surface of the effervescent solution. A bulk density greater than 0.6 g/ml leaves undissolved particles on the botom of the effervescent solution. In a preferred embodiment, the bulk density is from 0.25 to 0.5 g/ml. In a more preferred embodiment, the bulk density is from 0.35 to 0.47 g/ml. In a preferred embodiment, the therapeutic agent is present from 9 to 25% by weight. In a more preferred embodiment, the therapeutic agent is present from 12% to 20% by weight.

The granulating agent used in this invention can be any water soluble pharmaceutically acceptable granulating agent having a viscosity below 100 cps ($\triangleq$ 0,1 Pas) 10% by weight, at 25° in water, and being compatible with the therapeutic agent. The granulating agent may be selected from the group consisting of water, alcohol, polyvinylpyrrolidone, sucrose, hydroxypropyl cellulose and mixtures thereof. The preferred granulating agent for use in the instant invention is polyvinylpyrrolidone.

The granulating agent is present in the instant invention in amount from about 0.03 to 2.5% by weight. Less than 0.03% granulating agent causes a residue of surface film containing undissolved therapeutic agent to form. Greater than 2.5% causes slow disintegration. In a preferred embodiment, the granulating agent is present in an amount from 0.05 to 2.0% and more preferably in an amount from 0.08 to 0.23%.

The granulation formed by combining the therapeutic agent with granulating agent is ground and screened such that it has a particle size from 100 to 600 μm. A particle size of less than 100 μm yields a chalky material which results in processing problems such as poor mixing and compressibility properties. A particle size of greater than 600 microns causes a slow rate of solubilization and will leave undissolved therapeutic agent on the bottom of the effervescent solution after disintegration of the effervescent composition. In a preferred embodiment, the granulation has a particle size of 100 to 400 μm and more preferably has a particle size of 125 to 225 μm. In a preferred embodiment, the granulation has a particle size from 125 to 175 μm and acetaminophen is the therapeutic agent.

The effervescent system may comprise one or more components. The component of the effervescent system blended with the granulated therapeutic agent is in a microparticulate form having a particle size from 50 to 600 μm. In a preferred embodiment, the effervescent system comprises a carbonate containing material and an acid. The microparticulate component of the effervescent system may be the carbonate containing material, the acid and mixtures thereof.

In a preferred embodiment, the microparticulate component of the effervescent system is the acid. The microparticulate acid has a particle size of from 50 to 600 μm. A particle size of less than 50 μm promotes excessive static charge and results in processing problems such that the acid will not mix uniformly with the granulation and the mixture will not flow consistently. A particle size of greater than 600 μm causes slow disintegration. In a preferred embodiment, the microparticulate acid has a particle size of 50 to 275 μm and more preferably has a particle size of 75 to 175 μm. The microparticulate acid is present in an amount from 5 to 30% such that the total acid content of the effervescent composition is from 22 to 46% by weight. Microparticulate acid content of less than 5% results in slow disintegration and slow rate of solution of the therapeutic agent. Microparticulate acid content greater than 30% results in processing difficulties such as poor flow, and mixing as well as capping during tablet manufacture. In a preferred embodiment, the microparticulate acid is present in the amount of 7.5 to 22.5% by weight. In a more preferred embodiment, the microparticulate acid is present in the amount of 10 to 17%.

The acids which may be employed in practicing the present invention are compounds capable of reacting with carbonate containing materials to cause the release of carbon dioxide when contacted with sufficient water. Suitable acids include citric acid, fumaric acid, adipic acid, malic acid, tartaric acid, and the like, including mixtures thereof. The preferred acid is citric acid. In a preferred embodiment, the non-microparticulate acid has a particle size greater than 600 μm. This larger particle size is necessary to prevent manufacturing problems such as capping.

In the instant invention, the same acid is usually employed in the preblended mixture component and in the effervescent system, however, it is within the scope of the present invention for the two components to contain different acids including different mixtures of acids.

The granulated therapeutic agent and microparticulate acid are admixed to form the preblended mixture. The preblended mixture is present in the instant invention in an amount from 7 to 57.5% by weight. In a preferred embodiment the preblended mixture component is present from 20 to 35% by weight and more preferably is present from 25 to 30% by weight.

The effervescent system comprises all the ingredients of the rapid dissolving, effervescent composition except those contained in the preblended mixture. The effervescent system is present in an amount from 42.5 to 90% by weight.

The effervescent system may be selected from a wide variety of materials that are capable of producing effervescence in water. A particularly preferred method uses carbonate containing materials.

The carbonate containing materials which may be employed in practicing the present invention are compounds which are capable of reacting with acidic compounds with the release of carbon dioxide when contacted with sufficient water. In particular, the inorganic carbonates, and more particularly the alkali metal and ammonium carbonate materials may be used. Illustrative compounds include sodium carbonate, sodium bicarbonate, sodium sesquicarbonate, potassium carbonate, potassium bicarbonate, lithium carbonate, lithium bicarbonate, ammonium carbonate, ammonium bicarbonate, ammonium sesquicarbonate and the like, including mixtures of these.

The carbonate containing materials, when used, are present in an amount from about 4o to about 60% by weight of the total therapeutic effervescent composition.

The therapeutic effervescent composition may additionally contain conventional additives such as lubricants, antifoaming agents, flavoring agents, colorants, sweeteners and glidants.

The lubricant used in the present invention can be any pharmaceutically acceptable lubricant which includes metallic stearates, stearic acid, hydrogenated vegetable oils, polyethylene glycols, corn starch, sodium benzoate, sodium acetate and the like or mixtures thereof. Sodium benzoate is the preferred lubricant of the present invention. The lubricant is present in an amount from 1.7 to 10% by weight.

The antifoaming agent used in the present invention can be any pharmaceutically acceptable antifoaming agent which includes simethicone and the like, or mixtures thereof. Simethicone is the preferred antifoaming agent of the present invention. The antifoaming agent is present in an amount from 0.05 to 0.22% by weight.

Suitable flavoring agents include both natural and artificial flavors, and mints such as peppermint, menthol, artificial vanilla, cinnamon, various fruit flavors, both individual and mixed, and the like are contemplated. The flavorings are generally utilized in amounts that will vary depending upon the individual flavor, and may, for example, range in amounts of 0.5 to 3% by weight of the final composition weight.

In the instance where sweeteners are utilized, the present invention contemplates the inclusion of those sweeteners well known in the art, including both natural and artificial sweeteners. Thus, additional sweeteners may be chosen from the following non-limiting list: sugars such as sucrose, glucose (corn syrup), invert sugar, fructose, and mixtures thereof; saccharin and its various salts such as the sodium or calcium salt; cyclamic acid and its various salts such as the sodium salt; the dipeptide sweeteners such as aspartame; dihydrochalcone; glycyrrhizin; *Stevia rebaudiana* (Stevioside); and sugar alcohols such as sorbitol, sorbitol syrup, mannitol, xylitol, and the like. Also contemplated as an additional sweetener is the nonfermentable sugar substitute (hydrogenated starch hydrolysate) which is described in U.S. reissue patent 26,959. Also contemplated is the synthetic sweetener 3,6-dihydro-6-methyl-1-1-2,3-oxathiazin-4-one-2,2-dioxide particularly the potassium (Acesulfame-K), sodium and calcium salts thereof as described in German Patent No. 2,001,017.7. The sweeteners are used in amounts of up to 5% by weight. In general, the amount of sweetener will vary according to the type of sweetener and the desired taste of the final product. Natural sweeteneres are generally used in amounts up to about 5% by weight. In contrast, artificial sweeteners are used in amounts up to 1% by weight.

The colorants useful in the present invention include the pigments, such as titanium dioxide, that may be incorporated in amounts of up to 1% by weight, and preferably up to 0.6% by weight. Also, the colorants may include other dyes suitable for food, drug and cosmetic applications, and known as F.D. & C. dyes and the like. The materials acceptable for the foregoing spectrum of use are preferably water-soluble. Illustrative examples include indigoid dye, known as F.D. & C. Blue No. 2, which is the disodium salt of 5,5'-indigotin disulfonic acid. Simularly, the dye known as F.D. & C. Green No. 1, comprises a triphenylmethane dye and is the monosodium salt of 4-[4-N ethyl-p-sulfo-benzylamino)diphenylmethylene][1-(N-ethyl-N-p-sulfoniumbenzyl)-2,5-cyclohexadienimine]. A full recitation of all F.D. & C. and D. & C. dyes and their corresponding chemical structures may be found in the Kirk-Othmer Encyclopedia of Chemical Technology, in Volume 5, at Pages 857-884, which are suitable for use in the present invention.

In the instance where glidants are utilized, the present invention contemplates glidants such as microfine silicas, corn starch, microcrystalline cellulose, metallic stearates and the like, including mixtures of these that may be incorporated in amounts up to 2%.

A process for preparing the rapid dissolving therapeutic effervescent composition has also been unexpectedly discovered. The process comprises:

(A) forming a granulation by dissolving a solution and mixing the solution into the therapeutic agent;

(B) drying the granulation;

(C) sizing the dried granulation;

(D) admixing a microparticulate component of an effervescent system with the sized granulation of step (C) to form a mixture, and

(E) mixing an effervescent system with the mixture of step (D) to obtain a uniform mixture of granules and recovering the product.

More specifically, the process involves dissolving the granulating agent in an amount from 0.03 to 2.5% by weight in a solvent to form a solution containing from 1 to 50% by weight granulating agent, mixing the therapeutic agent with the solution of granulating agent and solvent to form a granulation, drying the granulation at between 50 to 70°C, sizing the dried granulation by grinding and screening to obtain particles having a size of 100 to 600 μm, admixing the microparticulate acid with a sized granulation; mixing the antifoaming agent, the carbonate containing material, the acid, the lubricant, the sweetener, and flavors to form a uniform effervescent system containing mixture; blending this effervescent system containing mixture with the granulation and microparticulate acid mixture and recovering the product. The final product may be used as is or formed into any desirable shape such as a tablet to render the product suitable for providing the necessary amount of therapeutic agent.

The present invention is further illustrated by the following examples. All parts and percentages in the examples and throughout the specification and claims are by weight of the total effervescent composition.

Example 1

Inventive Run 1

This Example demonstrates the formation of a product of this invention.
The following ingredients were admixed in accordance with the procedure listed below.

| Ingredient | Percent by Weight in the Final Product |
|---|---|
| Therapeutically Active Component | |
| Acetaminophen | 14.05 |
| Polyvinylpyrrolidone | 0.17 |
| Distilled Water (80 ml) | — |
| Citric Acid, microparticulate | 14.05 |
| Effervescent Component | |
| Sodium Bicarbonate | 47.77 |
| Simethicone | 0.14 |
| Distilled Water (5 ml) | — |
| Citric Acid | 14.05 |
| Sodium Carbonate | 4.78 |
| Sugar | 1.69 |
| Sodium Benzoate | 3.30 |

Dissolve the polyvinylpyrrolidone in distilled water (80 ml). Slowly add the polyvinylpyrrolidone solution to the acetaminophen while mixing. Dry the resultant granulation at 60°C until dried. Size the dried granulation through an oscillating granulator equipped with a No. 60 mesh screen (U.S. standard). Microparticulate citric acid (50 to 600 μm) was blended with the sized granulation.

Disperse the simethicone in 100 g of sodium bicarbonate.

Add distilled water (5 ml) to the remainder of the sodium bicarbonate while mixing, then add with continued mixing the citric acid, sodium carbonate, the active granulation-citric acid blend, the sugar, the simethicone-sodium bicarbonate -blend, and sodium benzoate and mix until a uniform blend is obtained. Compress tablets using flat faced beveled edged tooling to a weight of 3.56 g having a diameter of $^{15}/_{16}$ inches ($\triangleq$ 23,8 mm) and a hardness of 7-9 strong cobb hardness units.

Tablets made by the inventive procedure and the inventive formula described above disintegrated rapidly in 55 seconds with no undissolved drug residue when placed in 200ml of water at 22°C.

Example II

Inventive Run B and Comparative Runs A & C

This Example demonstrates the effect bulk density of the therapeutic agent has on the dissolution of a product prepared by the process and composition of Example 1. The same amount of acetaminophen as used in Example I was used in each product given below. The bulk density of the acetaminophen varies.

## FORMULATION

| Property | Comparative A | Inventive B | Comparative C |
|---|---|---|---|
| Bulk Density of Acetaminophen | 0.144 to 0.176 g/ml | 0.353 to 0.465 g/ml | 0.674 to 0.802 g/ml |
| Disintegration | 50 sec. | 55 sec. | 55 sec. |
| pH | 6.15 | 6.15 | 6.15 |
| Appearance | Clear solution powder on liquid surface | No powder on surface, clear solution, no un-dissolved drug | Clear solution, slight undissolved drug on bottom |
| Residue after 5 minutes | Slight residue on surface | Clear, no undissolved drug | Clear solution, slight residue of drug on bottom |

The results indicate that the product made by inventive formulation B with acetaminophen having a bulk density between 0.353 to 0.465 g/ml is the only product without undissolved drug after disintegration of the tablet and 5 minutes after the tablet was placed in water.

Product A was made with acetaminophen having a bulk density less than 0.2 g/ml. This resulted in undissolved drug floating on the surface of the solution.

Product C was made with acetaminophen having a bulk density greater than 0.6 g/ml. This results in undissolved drug on the bottom of the solution.

The disintegration test is conducted by placing 1 tablet into 200 ml of water at 22°C. Observations are made at the time of complete disintegration of the tablet and at 5 minutes after the start of the test.

Example III

Inventive Run 1 and Comparative Products D, E, F, G & H.

This Example demonstrates comparative results between the inventive composition and 5 similar commercial products after disintegration in 200 ml of water at 22°C.

| Product | Disintegration Time | pH | Post Disintegration Appearance | |
|---|---|---|---|---|
| | | | Initial | 5 Minutes |
| Inv. 1 | 55 sec. | 6.15 | Clear, no un-dissolved drug | Clear, no un-dissolved drug |
| Comp. D | 5 min. | 5.90 | Surface film, cloudy solution | Surface film, undissolved tablet sediment and drug |
| E | 75 sec. | 6.10 | Surface film, undissolved drug | Considerable un-dissolved drug |
| F | 75 sec. | 6.15 | Surface film, undissolved drug | Considerable un-dissolved drug |
| G | 90 sec. | 5.30 | Frothy, foaming, cloudy liquid | Cloudy liquid, no particulate material |
| H | 120 sec. | 6.05 | Clear solution, undissolved drug on surface | Clear solution, undissolved drug on surface |

The results clearly demonstrate that only the inventive composition 1, prepared by the formulation and procedure of Example I, results in a clear, palatable, effervescent solution at the end of disintegration and at 5 minutes after disintegration. Products D to H inclusive are commercially available products.

The presence of undissolved drug on the surface of the liquid or suspended throughout the liquid after distintegration of the tablet is considered to render the product unpalatable. All six products tested contained 500 mg of acetaminophen as the active ingredient. Distintegration was in 200 ml of water at 22°C.

Example IV

Inventive Runs 2, 3 & 4 and Comparative Runs I, J & K

This Example demonstrates the effect of acetaminophen/polyvinylpyrrolidone granulation particle size on the dissolution of a product prepared by the process and composition of Example I. The acetaminophen/ polyvinylpyrrolidone granulation of Example I was sized through U.S. standard mesh screens to produce granulation of various particle sizes.

| Product | U.S. Standard Mesh Size | Typical Particle Size | Results of Dissolution Test |
|---|---|---|---|
| Comparative | | | |
| I | 10 | 850 μm | Poor dissolution, undissolved drug present |
| J | 20 | 650 μm | Poor dissolution, undissolved drug present |
| K | 40 | 275 μm | Undissolved drug present |
| Inventive | | | |
| 2 | 60 | 175 μm | No undissolved drug good dissolution |
| 3 | 80 | 125 μm | No undissolved drug, good dissolution |
| 4 | 100 | 100 μm | No undissolved drug, good dissolution |

The results indicate that product made with acetaminophen/polyvinylpyrrolidone having a particle size corresponding to a 40 mesh U.S. standard screen (400 μm) or larger is unacceptable as it forms a product that does not completely dissolve. Granulation having a particle size less than 400 microns forms a product containing no undissolved drug.

Dissolution was carried out by placing 1 tablet into 200 ml of water at 22°C.

Example V

Inventive Runs 5 to 19 and Comparative Run L

This example demonstrates the effect of polyvinylpyrrolidone content on the dissolution of a product prepared by the process and composition of Example I.

| Product | Polyvinyl-pyrrolidone (%) | Results of Dissolution Test |
|---|---|---|
| **COMPARATIVE** | | |
| L | 0 | Undissolved particles on surface, poor drug dispersement |
| **INVENTIVE** | | |
| 5 | 0.028 | Acceptable dissolution, no undissolved drug |
| 6 | 0.056 | Good dissolution, no undissolved drug |
| 7 | 00844 | Same |
| 8 | 0.113 | Same |
| 9 | 0.141 | Same |
| 10 | 0.169 | Same |
| 11 | 0.197 | Same |
| 12 | 0.225 | Same |
| 13 | 0.253 | Same |
| 14 | 0.281 | Same |
| 15 | 0.309 | Same |
| 16 | 0.337 | Same |
| 17 | 0.671 | Same |
| 18 | 1.319 | Same |
| 19 | 2.068 | Disintegration time >1.5 min. No undissolved drug. |

The results indicate that product made without polyvinylpyrrolidone, comparative run L, does not completely solubilize and leaves undissolved particles on the surface of the solution after the dissolution test. The results further indicate that polyvinylpyrrolidone content in excess of 2.068% will cause extended disintegration times greater than 1.5 minutes.

Dissolution was carried out by placing 1 tablet into 200 ml of water at 22°C.

## Claims

**Claims for the following Contracting States : BE, CH, DE, FR, GB, IT, LI, LU, NL, SE**

1. A rapidly-dissolving therapeutic effervescent composition which comprises:
   (1) a pre-blended mixture of:
      (a) a granulated therapeutic agent having a particle size in the range of from 100 to 400μm; and
      (b) a component of a first effervescent system, said component having a particle size in the range of from 50 to 600μm; and
   (2) a second effervescent system, which may be the same as or different to the first effervescent system.

EP 0 203 768 B2

2. A composition according to Claim 1, wherein the pre-blended mixture is present in an amount of from 7 to 57.5% by weight of the final composition, and the second effervescent system is present in an amount of from 42.5 to 90% by weight of the effervescent composition.

3. A composition according to Claim 1 or 2, wherein the granulated therapeutic agent comprises a therapeutic agent and a granulating agent.

4. A composition according to Claim 3, wherein the granulating agent is characterized by:
(a) being water soluble.
(b) having a viscosity below 100 cps ($\triangleq$ 0,1 Pas), 10% by weight, at 25°C in water; and
(c) being compatible with the therapeutic agent.

5. A composition according to Claim 3 or 4, wherein the granulating agent is present in an amount of from 0.03 to 2.5% by weight of the effervescent composition.

6. A composition according to any one of Claims 3 to 5, wherein the granulating agent is chosen from: water, alcohol, polyvinylpyrrolidone, sucrose, hydroxypropyl celulose and mixtures thereof, the granulating agent preferably being polyvinylpyrrolidone.

7. A composition according to any one of Claims 3 to 6, wherein the therapeutic agent is present in an amount of from 2 to 27% by weight of the effervescent composition.

8. A composition according to any one of Claims 3 to 7, wherein the therapeutic agent is an analgesic chosen from aspirin, acetaminophen, ibuprofen and mixtures thereof, the analgesic preferably being acetaminophen having a bulk density in the range of from 0.2 to 0.6 g/ml, and preferably having a particle size in the range of from 100 to 400 $\mu$m.

9. A composition according to any preceding claim, wherein the component of the first effervescent system blended with the granulated therapeutic agent is present in an amount of from 5 to 30% by weight of the effervescent composition.

10. A composition according to any preceding claim, wherein the second effervescent system is characterized by releasing a non-toxic gas when contacted with water and by being compatible with the therapeutic agent.

11. A composition according to any preceding claim, wherein the second effervescent system comprises:
a carbonate containing material preferably chosen from sodium carbonate, sodium bicarbonate, sodium sesquicarbonate, potassium carbonate, potassium bicarbonate, lithium carbonate, lithium bicarbonate, ammonium carbonate, ammonium bicarbonate, ammonium sesquicarbonate and mixtures thereof; and
an acid, preferably chosen from citric acid, fumaric acid, adipic acid, malic acid, tartaric acid and mixtures thereof, more preferably the acid being citric acid.

12. A composition according to Claim 11, wherein the carbonate containing material is present in an amount of from 40 to 60% by weight of the effervescent composition.

13. A composition according to any preceding claim, wherein the component of the first effervescent system in the pre-blended mixture is an acid having a particle size of from 50 to 600 $\mu$m, preferably chosen from citric acid, fumaric acid, adipic acid, malic acid, tartaric acid and mixtures thereof, more preferably the acid being citric acid.

14. A composition according to Claim 13 when appendant to Claim 12 in which the first and second effervescent systems are the same and wherein said particulate acid of the first effervescent system is present in an amount of from 5 to 30% by weight of the effervescent composition such that the total acid content of the effervescent composition is in the range of from 22 to 46% by weight.

15. A rapidly-dissolving therapeutic effervescent composition which comprises:
(1) a pre-blended mixture present in an amount of from 7 to 57.5% of:
(a) a granulated therapeutic agent having a particle size in the range of from 100 to 400$\mu$m of
(i) a therapeutic agent present in an amount of from 2 to 27%; and

11

(ii) a granulating agent present in an amount of from 0.03 to 2.5%; and

(b) an acid having a particle size in the range of from 50 to 600μm and being present in an amount of from 5 to 30%; and

(2) an effervescent system present in an amount of from 42.5 to 90% and comprising:

(c) a carbonate containing material in an amount of from 40 to 60%; and

(d) an acid in an amount of from 2.5 to 30%; wherein the total acid content of parts (b) and (d) above is in the range of from 22 to 46%; all percentages being by weight based on the weight of the effervescent composition.

16. A composition according to Claim 15 having any one or more of the features of Claims 4, 6 and 8.

17. A composition according to Claim 15 or 16, wherein the acid of parts (b) and (d) is chosen from citric acid, fumaric acid, adipic acid, malic acid, tartaric acid and mixtures thereof; the acid preferably being citric acid.

18. A composition according to Claim 15, 16 or 17, wherein the carbonate containing material is chosen from sodium carbonate, sodium bicarbonate, sodium sesquicarbonate, potassium carbonate, potassium bicarbonate, lithium carbonate, lithium bicarbonate, ammonium carbonate, ammomium bicarbonate, ammonium sesquicarbonate and mixtures thereof.

19. A method for preparing a rapidly dissolving effervescent composition of a therapeutic agent, which method comprises:

(1) forming a granulation by dissolving a granulating agent in a solvent to form a solution containing in the range of from 1 to 50% by weight of the granulating agent, and mixing a therapeutic agent with the solution;

(2) drying the granulation,

(3) sizing the dried granulation by grinding and screening to obtain particles having a size in the range of from 100 to 400μm;

(4) admixing a component of a first effervescent system having a particle size in the range of from 50 to 600μm with the sized granulation of step (3) to form a pre-blended mixture;

(5) mixing a second effervescent system, which may be the same as or different to the first effervescent system, with the pre-blended mixture of step (4) to obtain a uniform mixture of granules; and

(6) recovering the product.

20. A method as claimed in Claim 19, wherein the component of the first effervescent system in step (4) is an acid.

21. A method according to Claim 19 or 20, wherein the therapeutic agent is chosen from: acetaminophen, aspirin, ibuprofen and mixtures thereof; preferably acetaminophen having a bulk density of from 0.2 to 06 g/ml.

22. A method according to any one of Claims 19 to 22, further comprising the step of forming the mixture of step (5) into a tablet.

**Claims for the following Contracting State : AT**

1. A method for preparing a rapidly-dissolving therapeutic effervescent composition which method comprises combining together:

(1) a pre-blended mixture of:

(a) a granulated therapeutic agent having a particle in the range of from 100 to 400 μm; and

(b) a component of a first effervescent system, said component having a particle size in the range of from 50 to 600 μm; and

(2) a second effervescent system, which may be the same as or different to the first effervescent system.

2. A method according to Claim 1, wherein the pre-blended mixture is present in an amount of from 7 to 57.5% by weight of the final composition, and the second effervescent system is present in an amount of from 42.5 to 90% by weight of the effervescent composition.

3. A method according to Claim 1 or 2, wherein the granulated therapeutic agent comprises a therapeutic agent and a granulating agent.

4. A method according to Claim 3, wherein the granulating agent is characterized by:
   (a) being water soluble;
   (b) having a viscosity below 100 cps ($\hat{=}$ 0,1 Pas), 10% by weight, at 25°C in water; and
   (c) being compatible with the therapeutic agent.

5. A method according to Claim 3 or 4, wherein the granulating agent is present in an amount of from 0.03 to 2.5% by weight of the effervescent composition.

6. A method according to any one of Claims 3 to 5, wherein the granulating agent is chosen from: water, alcohol, polyvinylpyrrolidone, sucrose, hydroxypropyl celulose and mixtures thereof, the granulating agent preferably being polyvinylpyrrolidone.

7. A method according to any one of Claims 3 to 6, wherein the therapeutic agent is present in an amount of from 2 to 27% by weight of the effervescent composition.

8. A method according to any one of Claims 3 to 7, wherein the therapeutic agent is an analgesic chosen from aspirin, acetaminophen, ibuprofen and mixtures thereof, the analgesic preferably being acetaminophen having a bulk density in the range of from 0.2 to 0.6 g/ml, and preferably having a particle size in the range of from 100 to 400 $\mu$m.

9. A method according to any preceding claim, wherein the component of the first effervescent system blended with the granulated therapeutic agent is present in an amount of from 5 to 30% by weight of the effervescent composition.

10. A method according to any preceding claim, wherein the second effervescent system is characterized by releasing a non-toxic gas when contacted with water and by being compatible with the therapeutic agent.

11. A method according to any preceding claim, wherein the second effervescent system comprises:
   a carbonate containing material preferably chosen from sodium carbonate, sodium bicarbonate, sodium sesquicarbonate, potassium carbonate, potassium bicarbonate, lithium carbonate, lithium bicarbonate, ammonium carbonate, ammonium bicarbonate, ammonium sesquicarbonate and mixtures thereof; and
   an acid, preferably chosen from citric acid, fumaric acid, adipic acid, malic acid, tartaric acid and mixtures thereof, more preferably the acid being citric acid.

12. A method according to Claim 11, wherein the carbonate containing material is present in an amount of from 40 to 60% by weight of the effervescent composition.

13. A method according to any preceding claim, wherein the component of the first effervescent system in the pre-blended mixture is an acid having a particle size of from 50 to 600 $\mu$m, preferably chosen from citric acid, fumaric acid, adipic acid, malic acid, tartaric acid and mixtures thereof, more preferably the acid being citric acid.

14. A method according to Claim 13 when appendant to Claim 12 in which the first and second effervescent systems are the same and wherein said particulate acid of the first effervescent system is present in an amount of from 5 to 30% by weight of the effervescent composition such that the total acid content of the effervescent composition is in the range of from 22 to 46% by weight.

15. A method of preparing a rapidly-dissolving therapeutic effervescent composition which method comprises combining together:
   (1) a preblended mixture present in an amount of from 7 to 57.5% of:
      (a) a granulated therapeutic agent having a particle size in the range of from 100 to 400 $\mu$m of
         (i) a therapeutic agent present in an amount of from 2 to 27%; and
         (ii) a granulating agent present in an amount of from 0.03 to 2.5%; and
      (b) an acid having a particle size in the range of from 50 to 600 $\mu$m and being present in an amount of from 5 to 30%; and
   (2) an effervescent system present in an amount of from 42.5 to 90% and comprising:
      (c) a carbonate containing material in an amount of from 40 to 60%; and

13

(d) an acid in an amount of from 2.5 to 30%; wherein the total acid content of parts (b) and (d) above is in the range of from 22 to 46%; all percentages being by weight based on the weight of the effervescent composition.

**16.** A method according to Claim 15 having any one or more of the features of Claims 4, 6 and 8.

**17.** A method according to Claim 15 or 16, wherein the acid of parts (b) and (d) is chosen from citric acid, fumaric acid, adipic acid, malic acid, tartaric acid and mixtures thereof; the acid preferably being citric acid.

**18.** A method according to Claim 15, 16 or 17, wherein the carbonate containing material is chosen from sodium carbonate, sodium bicarbonate, sodium sesquicarbonate, potassium carbonate, potassium bicarbonate, lithium carbonate, lithium bicarbonate, ammonium carbonate, ammonium bicarbonate, ammonium sesquicarbonate and mixtures thereof.

**19.** A method for preparing a rapidly dissolving effervescent composition of a therapeutic agent, which method comprises:
  (1) forming a granulation by dissolving a granulating agent in a solvent to form a solution containing in the range of from 1 to 50 % by weight of the granulating agent, and mixing a therapeutic agent with the solution;
  (2) drying the granulation,
  (3) sizing the dried granulation by grinding and screening to obtain particles having a size in the range of from 100 to 400 $\mu$m;
  (4) admixing a component of a first effervescent system having a particle size in the range of from 50 to 600 $\mu$m with the sized granulation of step (3) to form a pre-blended mixture;
  (5) mixing a second effervescent system (which may be the same as or different to the first effervescent system) with the pre-blended mixture of step (4) to obtain a uniform mixture of granules; and
  (6) recovering the product.

**20.** A method as claimed in Claim 19, wherein the component of the first effervescent system in step (4) is an acid.

**21.** A method according to Claim 19 or 20, wherein the therapeutic agent is chosen from: acetaminophen, aspirin, ibuprofen and mixtures thereof; preferably acetaminophen having a bulk density of from 0.2 to 0.6 g/ml.

**22.** A method according to any one of Claims 19 to 22, further comprising the step of forming the mixture of step (5) into a tablet.

**Patentansprüche**

**Patentansprüche für folgende Vertragsstaaten : BE, CH, DE, FR, GB, IT, LI, LU, NL, SE**

**1.** Schnellösliche therapeutische Brausezusammensetzung, umfassend:
  (1) eine vorvermengte Mischung von:
    (a) einem granulierten therapeutischen Mittel, welches eine Teilchengröße im Bereich von 100 bis 400 $\mu$m aufweist; und
    (b) einem Bestandteil eines ersten Brausesystems, welcher Bestandteil eine Teilchengröße im Bereich von 50 bis 600 $\mu$m aufweist; und
  (2) ein zweites Brausesystem, welches das gleiche wie das erste oder ein anderes als das erste Brausesystem sein kann.

**2.** Zusammensetzung nach Anspruch 1, worin die vorvermengte Mischung in einer Menge von 7 bis 57,5 Gew.-% der Endzusammensetzung anwesend ist, und das zweite Brausesystem in einer Menge von 42,5 bis 90 Gew.-% der Brausezusammensetzung anwesend ist.

**3.** Zusammensetzung nach Anspruch 1 oder 2, worin das granulierte therapeutische Mittel ein therapeutisches Mittel und ein Granulierungsmittel umfaßt.

4. Zusammensetzung nach Anspruch 3, worin das Granulierungsmittel dadurch gekennzeichnet ist, daß es
(a) wasserlöslich ist,
(b) eine Viskosität unter 100 cp = 0,1 Pa·s, 10 Gew.-%, bei 25°C in Wasser aufweist; und
(c) mit dem therapeutischen Mittel kompatibel ist.

5. Zusammensetzung nach Anspruch 3 oder 4, worin das Granulierungsmittel in einer Menge von 0,03 bis 2,5 Gew.-% der Brausezusammensetzung anwesend ist.

6. Zusammensetzung nach irgendeinem der Ansprüche 3 bis 5, worin das Granulierungsmittel ausgewählt ist aus: Wasser, Alkohol, Polyvinylpyrrolidon, Saccharose, Hydroxypropylcellulose und Mischungen davon, welches Granulierungsmittel vorzugsweise Polyvinylpyrrolidon ist.

7. Zusammensetzung nach irgendeinem der Anspruch 3 bis 6, worin das therapeutische Mittel in einer Menge von 2 bis 27 Gew.-% der Brausezusammensetzung anwesend ist.

8. Zusammensetzung nach irgendeinem der Ansprüche 3 bis 7, worin das therapeutische Mittel ein Analgetikum ausgewählt aus Aspirin, Acetaminophen, Ibuprofen und Mischungen davon ist, welches Analgetikum vorzugsweise Acetaminophen mit einer Schüttdichte im Bereich von 0,2 bis 0,6 g/ml und vorzugsweise mit einer Teilchengröße im Bereich von 100 bis 400 μm ist.

9. Zusammensetzung nach irgendeinem vorhergehenden Anspruch, worin der mit dem granulierten therapeutischen Mittel vermengte Bestandteil des ersten Brausesystems in einer Menge von 5 bis 30 Gew.-% der Brausezusammensetzung anwesend ist.

10. Zusammensetzung nach irgendeinem vorhergehenden Anspruch, worin das zweite Brausesystem dadurch gekennzeichnet ist, daß es bei Kontakt mit Wasser ein nicht-toxisches Gas freisetzt, und daß es mit dem therapeutischen Mittel kompatibel ist.

11. Zusammensetzung nach irgendeinem vorhergehenden Anspruch, worin das zweite Brausesystem umfaßt: ein Carbonat enthaltendes Material, welches vorzugsweise ausgewählt ist aus: Natriumcarbonat, Natriumbicarbonat, Natriumsesquicarbonat, Kaliumcarbonat, Kaliumbicarbonat, Lithiumcarbonat, Lithiumbicarbonat, Ammoniumcarbonat, Ammoniumbicarbonat, Ammoniumsesquicarbonat und Mischungen davon; und
eine Säure, welche vorzugsweise ausgewählt ist aus: Zitronensäure, Fumarsäure, Adipinsäure, Äpfelsäure, Weinsäure und Mischungen davon, welche Säure insbesondere Zitronensäure ist.

12. Zusammensetzung nach Anspruch 11, worin das Carbonat enthaltende Material in einer Menge von 40 bis 60 Gew.-% der Brausezusammensetzung anwesend ist.

13. Zusammensetzung nach irgendeinem vorhergehenden Anspruch, worin der Bestandteil des ersten Brausesystems in der vorvermengten Mischung eine Säure ist, welche eine Teilchengröße von 50 bis 600 μm aufweist, vorzugsweise ausgewählt ist aus: Zitronensäure, Fumarsäure, Adipinsäure, Äpfelsäure, Weinsäure und Mischungen davon, welche Säure insbesondere Zitronensäure ist.

14. Zusammensetzung nach Anspruch 13, wenn auf Anspruch 12 rückbezogen, worin das erste und das zweite Brausesystem dasselbe ist, und worin diese aus Partikeln bestehende Säure des ersten Brausesystems in einer Menge von 5 bis 3o Gew.-% der Brausezusammensetzung anwesend ist, so daß der Gesamtsäuregehalt der Brausezusammensetzung im Bereich von 22 bis 46 Gew.-% liegt.

15. Schnellösliche therapeutische Brausezusammensetzung, umfassend:
(1) eine vorvermengte Mischung in einer Menge von 7 bis 57,5 % von:
(a) einem granulierten therapeutischen Mittel mit einer Teilchengröße im Bereich von 100 bis 400 μm von
(i) einem therapeutischen Mittel in einer Menge von 2 bis 27 %; und
(ii) einem Granulierungsmittel in einer Menge von 0,03 bis 2,5 %; und
(b) einer Säure mit einer Teilchengröße im Bereich von 50 bis 600 μm in einer Menge von 5 bis 30 %; und
(2) einem Brausesystem in einer Menge von 42,5 bis 90 %, enthaltend:
(c) ein carbonathältiges Material in einer Menge von 40 bis 60 %; und

15

(d) eine Säure in einer Menge von 2,5 bis 30 %; worin der Gesamtsäuregehalt der obigen Teile (b) und (d) im Bereich von 22 bis 46 % liegt; wobei sämtliche Prozentangaben in Gewicht auf das Gewicht der Brausezusammensetzung bezogen sind.

16. Zusammensetzung nach Anspruch 15, welche irgendeines oder mehrere der Charakteristika der Ansprüche 4, 6 und 8 aufweist.

17. Zusammensetzung aus Anspruch 15 oder 16, worin die Säure der Teile (b) und (d) ausgewählt ist aus: Zitronensäure, Fumarsäure, Adipinsäure, Äpfelsäure, Weinsäure und Mischungen davon; welche Säure insbesondere Zitronensäure ist.

18. Zusammensetzung nach Anspruch 15, 16 oder 17, worin das Carbonat enthaltende Material ausgewählt ist aus: Natriumcarbonat, Natriumbicarbonat, Natriumsesquicarbonat, Kaliumcarbonat, Kaliumbicarbonat, Lithiumcarbonat, Lithiumbicarbonat, Ammoniumcarbonat, Ammoniumbicarbonat, Ammoniumsesquicarbonat und Mischungen davon.

19. Verfahren zur Herstellung einer schnellöslichen Brausezusammensetzung aus einem therapeutischen Mittel, welches Verfahren umfaßt:
    (1) die Bildung einer Granulation durch Lösen eines Granulationsmittels in einem Lösungsmittel zur Bildung einer Lösung, die das Granulierungsmittel in einem Bereich von 1 bis 50 Gew.% enthält, und Vermischen eines therapeutischen Mittels mit der Lösung;
    (2) Trocknen der Granulation,
    (3) Kalibrieren der getrockneten Granulation durch Zerkleinern und Sieben zum Erhalt von Teilchen mit einer Größe im Bereich von 100 bis 400 $\mu$m;
    (4) Zumischen eines Bestandteiles eines ersten Brausesystems mit einer Teilchengröße im Bereich von 50 bis 600 $\mu$m zur kalibrierten Granulation aus Schritt (3) zwecks Bildung einer vorvermengten Mischung;
    (5) Mischen eines zweiten Brausesystems, welches das gleiche wie das erste oder ein anderes als das erste Brausesystem sein kann, mit der vorvermengten Mischung aus Schritt (4) zum Erhalt einer einheitlichen Mischung von Granula; und
    (6) Gewinnung des Produkts.

20. Verfahren wie im Anspruch 19 beansprucht, worin der Bestandteil des ersten Brausesystems in Schritt (4) eine Säure ist.

21. Verfahren nach Anspruch 19 oder 20, worin das therapeutische Mittel ausgewählt ist aus: Acetaminophen, Aspirin, Ibuprofen und Mischungen davon; vorzugsweise Acetaminophen mit einer Schüttdichte von 0,2 bis 0,6 g/ml.

22. Verfahren nach irgendeinem der Ansprüche 19 bis 22, welches weiters den Schritt der Formung der Mischung aus Schritt (5) zu einer Tablette umfaßt.

**Patentansprüche für folgenden Vertragsstaat : AT**

1. Verfahren zur Herstellung einer schnell-löslichen therapeutischen Brausezusammensetzung, welches Verfahren das Vereinigen von:
    (1) einer vorvermengten Mischung von:
        (a) einem granulierten therapeutischen Mittel, welches eine Teilchengröße im Bereich von 100 bis 600 $\mu$m aufweist; und
        (b) einem Bestandteil eines ersten Brausesystems, welcher Bestandteil eine Teilchengröße im Bereich von 50 bis 600 $\mu$m aufweist; und
    (2) einem zweiten Brausesystem, welches dasselbe wie das erste Brausesystem sein kann oder sich von ihm unterscheiden kann, umfaßt.

2. Verfahren nach Anspruch 1, worin die vorvermengte Mischung in einer Menge von 7 bis 57,5 Gew.-% der Endzusammensetzung anwesend ist, und das zweite Brausesystem in einer Menge von 42,5 bis 90 Gew.-% der Bruasezusammensetzung anwesend ist.

3. Verfahren nach Anspruch 1 oder 2, worin das granulierte therapeutische Mittel ein therapeutisches Mittel und ein Granulierungsmittel umfaßt.

4. Verfahren nach Anspruch 3, worin das Granulierungsmittel dadurch gekennzeichnet ist, daß es
   (a) wasserlöslich ist,
   (b) eine Viskosität unter 100 cp = 0,1 Pa·s, 10 Gew.-%, bei 25°C in Wasser aufweist; und
   (c) mit dem therapeutischen Mittel kompatibel ist.

5. Verfahren nach Anspruch 3 oder 4, worin das Granulierungsmittel in einer Menge von 0,03 bis 2,5 Gew.-% der Brausezusammensetzung anwesend ist.

6. Verfahren nach irgendeinem der Ansprüche 3 bis 5, worin das Granulierungsmittel ausgewählt ist aus: Wasser, Alkohol, Polyvinylpyrrolidon, Saccharose, Hydroxypropylcellulose und Mischungen davon, welches Granulierungsmittel vorzugsweise Polyvinylpyrrolidon ist.

7. Verfahren nach irgendeinem der Anspruch 3 bis 6, worin das therapeutische Mittel in einer Menge von 2 bis 27 Gew.-% der Brausezusammensetzung anwesend ist.

8. Verfahren nach irgendeinem der Ansprüche 3 bis 7, worin das therapeutische Mittel ein Analgetikum ausgewählt aus Aspirin, Acetaminophen, Ibuprofen und Mischungen davon ist, welches Analgetikum vorzugsweise Acetaminophen mit einer Massendichte im Bereich von 0,2 bis 0,6 g/ml und vorzugsweise mit einer Teilchengröße im Bereich von 100 bis 400 µm ist.

9. Verfahren nach irgendeinem vorhergehenden Anspruch, worin der mit dem granulierten therapeutischen Mittel vermengte Bestandteil des ersten Brausesystems in einer Menge von 5 bis 30 Gew.-% der Brausezusammensetzung anwesend ist.

10. Verfahren nach irgendeinem vorhergehenden Anspruch, worin das zweite Brausesystem dadurch gekennzeichnet ist, daß es bei Kontakt mit Wasser ein nicht-toxisches Gas freisetzt, und daß es mit dem therapeutischen Mittel kompatibel ist.

11. Verfahren nach irgendeinem vorhergehenden Anspruch, worin das zweite Brausesystem umfaßt: ein Carbonat enthaltendes Material, welches vorzugsweise ausgewählt ist aus: Natriumcarbonat, Natriumbicarbonat, Natriumsesquicarbonat, Kaliumcarbonat, Kaliumbicarbonat, Lithiumcarbonat, Lithiumbicarbonat, Ammoniumcarbonat, Ammoniumbicarbonat, Ammoniumsesquicarbonat und Mischungen davon; und eine Säure, welche vorzugsweise ausgewählt ist aus: Zitronensäure, Fumarsäure, Adipinsäure, Apfelsäure, Weinsäure und Mischungen davon, welche Säure insbesondere Zitronensäure ist.

12. Verfahren nach Anspruch 11, worin das Carbonat enthaltende Material in einer Menge von 40 bis 60 Gew.-% der Brausezusammensetzung anwesend ist.

13. Verfahren nach irgendeinem vorhergehenden Anspruch, worin der Bestandteil des ersten Brausesystems in der vorvermengten Mischung eine Säure ist, welche eine Teilchengröße von 50 bis 600 µm aufweist, vorzugsweise ausgewählt ist aus: Zitronensäure, Fumarsäure, Adipinsäure, Apfelsäure, Weinsäure und Mischungen davon, welche Säure insbesondere Zitronensäure ist.

14. Verfahren nach Anspruch 13, wenn auf Anspruch 12 rückbezogen, worin das erste und das zweite Brausesystem dasselbe ist, und worin diese aus Partikeln bestehende Säure des ersten Brausesystems in einer Menge von 5 bis 30 Gew.-% der Brausezusammensetzung anwesend ist, sodaß der Gesamtsäuregehalt der Brausezusammensetzung im Bereich von 22 bis 46 Gew.-% liegt.

15. Verfahren zur Herstellung einer schnell-löslichen therapeutischen Brausezusammensetzung, welches Verfahren die Kombination von:
    (1) einer vorvermengten Mischung, welche anwesend ist in einer Menge von 7 bis 57,5% von:
        (a) einem granuliertem therapeutischen Mittel mit einer Teilchengröße im Bereich von 100 bis 600 µm von
            (i) einem therapeutischen Mittel, welches in einer Menge von 2 bis 27% anwesend ist; und
            (ii) einem Granulierungsmittel, welches in einer Menge von 0,03 bis 2,5% anwesend ist; und
        (b) einer Säure mit einer Teilchengröße im Bereich von 50 bis 600 µm, und welche in einer Menge von 5 bis 30% anwesend ist; und
    (2) eines Brausesystems, welches in einer Menge von 42,5 bis 90% anwesend ist und umfaßt:
        (c) ein Carbonat enthaltendes Material in einer Menge von 40 bis 60%; und

17

(d) eine Säure in einer Menge von 2,5 bis 30%;
worin der Gesamtsäuregehalt der Teile (b) und (d) oben im Bereich von 22 bis 46% liegt; wobei sich alle Gewichtsprozentangaben auf das Gewicht der Brausezusammensetzung beziehen, umfast.

16. Verfahren nach Anspruch 15, welches irgendeines oder mehrere der Charakteristika der Ansprüche 4, 6 und 8 aufweist.

17. Verfahren nach Anspruch 15 oder 16, worin die Säure der Teile (b) und (d) ausgewählt ist aus: Zitronensäure, Fumarsäure, Adipinsäure, Apfelsäure, Weinsäure und Mischungen davon; welche Säure insbesondere Zitronensäure ist.

18. Verfahren nach Anspruch 15, 16 oder 17, worin das Carbonat enthaltende Material ausgewählt ist aus: Natriumcarbonat, Natriumbicarbonat, Natriumsesquicarbonat, Kaliumcarbonat, Kaliumbicarbonat, Lithiumcarbonat, Lithiumbicarbonat, Ammoniumcarbonat, Ammoniumbicarbonat, Ammoniumsesquicarbonat und Mischungen davon.

19. Verfahren zur Herstellung einer schnell-löslichen Brausezusammensetzung eines therapeutischen Mittels, welches Verfahren umfaßt:
(1) Bildung einer Granulierung durch Lösen eines Granulierungsmittels in einem Lösungsmittel zur Bildung einer Lösung, welche das Granulierungsmittel im Bereich von 1 bis 50 Gew.-% enthält, und Vermischen eines therapeutischen Mittels mit der Lösung;
(2) Trocknen der Granulierung,
(3) Kalibrieren der getrockneten Granulierung durch Zerkleinern und Sieben zum Erhalt von Teilchen mit einer Größe im Bereich von 100 bis 600 μm;
(4) Vermischen eines Bestandteils eines ersten Brausesystems mit einer Teilchengröße im Bereich von 50 bis 600 μm mit der kalibrierten Granulation aus Schritt (3) zur Bildung einer vorvermengten Mischung;
(5) Mischen eines zweiten Brausesystems (welches dasselbe wie das erste sein kann oder verschieden davon sein kann) mit der vorvermengten Mischung aus Schritt (4) zum Erhalt einer einheitlichen Mischung von Granulaten; und
(6) Gewinnung des Produkts.

20. Verfahren wie im Anspruch 19 beansprucht, worin der Bestandteil des ersten Brausesystems in Schritt (4) eine Säure ist.

21. Verfahren nach Anspruch 19 oder 20, worin das therapeutische Mittel ausgewählt ist aus: Acetaminophen, Aspirin, Ibuprofen und Mischungen davon; vorzugsweise Acetaminophen mit einer Massendichte von 0,2 bis 0,6 g/ml.

22. Verfahren nach irgendeinem der Ansprüche 19 bis 22, welches weiters den Schritt der Formung der Mischung aus Schritt (5) zu einer Tablette umfaßt.

## Revendications

**Revendications pour les Etats contractants suivants : BE, CH, DE, FR, GB, IT, LI, LU, NL, SE**

1. Une composition thérapeutique effervescente à dissolution rapide qui comprend:
(1) un prémélange de:
(a) un agent thérapeutique sous forme de granules ayant une dimension particulaire comprise entre 100 et 400 μm; et
(b) un composé d'un premier système effervescent, ce composé ayant une dimension particulaire comprise entre 50 et 600 μm; et
(2) un second système effervescent, qui peut être identique ou différent du premier système effervescent.

2. Une composition selon la revendication 1, dans laquelle le prémélange est présent dans une proportion de 7 à 57,5% en poids de la composition finale et le système effervescent est présent dans une proportion de 42,5 à 90% en poids de la composition effervescente.

3. Une composition selon la revendication 1 ou 2, dans laquelle l'agent thérapeutique sous forme de granules comprend un agent thérapeutique et un agent de granulation.

4. Une composition selon la revendication 3, dans laquelle l'agent de granulation est caractérisé en ce que:
   (a) il est soluble dans l'eau;
   (b) il a une viscosité inférieure à 100 cps (0,1 Pa.s), à 10% en poids dans de l'eau à 25°C; et
   (c) il est compatible avec l'agent thérapeutique.

5. Une composition selon la revendication 3 ou 4, dans laquelle l'agent de granulation est présent dans une proportion de 0,03 à 0,25% en poids de la composition effervescente.

6. Une composition selon l'une quelconque des revendications 3 à 5, dans laquelle l'agent de granulation est choisi parmi: eau, alcool, polyvinylpyrrolidone, saccharose, hydroxypropylcellulose et les mélanges de ceux-ci, l'agent de granulation étant la polyvinylpyrrolidone.

7. Une composition selon l'une quelconque des revendications 3 à 6, dans laquelle l'agent thérapeutique est présent dans une proportion de 2 à 27% en poids de la composition effervescente.

8. Une composition selon l'une quelconque des revendications 3 à 7, dans laquelle l'agent thérapeutique est un analgésique choisi parmi: aspirine, acétaminophène, ibuprofène et les mélanges de ceux-ci, l'analgésique étant de préférence l'acétaminophène ayant une masse volumique comprise entre 0,2 et 0,6 g/ml et, de préférence, ayant une dimension particulaire comprise entre 100 et 400 μm.

9. Une composition selon l'une quelconque des revendications précédentes, dans laquelle le composé du premier système effervescent mélangé avec l'agent thérapeutique sous forme de granules est présent dans une proportion de 5 à 30% en poids de la composition effervescente.

10. Une composition selon l'une quelconque des revendications précédentes, dans laquelle le second système effervescent est caractérisé par le dégagement d'un gaz non toxique quand il est mis en contact avec l'eau et par le fait qu'il est compatible avec l'agent thérapeutique.

11. Une composition selon l'une quelconque des revendications précédentes, dans laquelle le second système effervescent comprend:
   - un composé contenant du carbonate de préférence choisi parmi: carbonate de sodium, bicarbonate de sodium, sesquicarbonate de sodium, carbonate de potassium, bicarbonate de potassium, carbonate de lithium, bicarbonate de lithium, carbonate d'ammonium, bicarbonate d'ammonium, sesquicarbonate d'ammonium et les mélanges de ceux-ci; et
   - un acide, de préférence choisi parmi: acide citrique, acide fumarique, acide adipique, acide malique, acide tartrique et les mélanges de ceux-ci, l'acide préféré étant l'acide citrique.

12. Une composition selon la revendication 11, dans laquelle le composé contenant du carbonate est présent dans une proportion de 40 à 60% en poids de la composition effervescente.

13. Une composition selon l'une quelconque des revendications précédentes, dans laquelle le composé du premier système effervescent dans le prémélange est un acide ayant une dimension particulaire comprise entre 50 et 600 μm, de préférence choisi parmi: acide citrique, acide fumarique, acide adipique, acide malique, acide tartrique et les mélanges de ceux-ci, l'acide préféré étant l'acide citrique.

14. Une composition selon la revendication 13, en combinaison avec la revendication 12, dans laquelle le premier et le second systèmes effervescents sont identiques, et dans laquelle ledit acide particulaire du premier système effervescent est présent dans une proportion de 5 à 30% en poids de la composition effervescente de telle sorte que la teneur totale en acide de la composition effervescente soit comprise entre 22 et 46% en poids.

15. Une composition thérapeutique effervescente à dissolution rapide qui comprend:
   (1) un prémélange, présent dans une proportion de 7 à 57,5% de:
      (a) un agent thérapeutique sous forme de granules ayant une dimension particulaire comprise entre 100 et 400 μm comprenant:
         (i) un agent thérapeutique présent dans une proportion de 2 à 27%; et
         (ii) un agent de granulation présent dans une proportion de 0,03 à 2,5%; et

(b) un acide ayant une dimension particulaire comprise entre 50 et 600 μm et étant présent dans une proportion de 5 à 30%; et

(2) un système effervescent présent dans une proportion de 42,5 à 90% et comprenant:

(c) un composé contenant du carbonate dans une proportion de 40 à 60%; et

(d) un acide dans une proportion de 2,5 à 30%, dans laquelle la teneur totale en acide des parties (b) et (d) précitées est comprise entre 22 et 46%, tous les pourcentages étant exprimés en poids par rapport au poids de la composition effervescente.

16. Une composition selon la revendication 15, ayant une quelconque ou plusieurs caractéristiques des revendications 4, 6 et 8.

17. Une composition selon la revendication 15 ou 16, dans laquelle l'acide des parties (b) et (d) est choisi parmi: acide citrique, acide fumarique, acide adipique, acide malique, acide tartrique et les mélanges de ceux-ci, l'acide préféré étant l'acide citrique.

18. Une composition selon la revendication 15, 16 ou 17, dans laquelle le composé contenant du carbonate est choisi parmi: carbonate de sodium, bicarbonate de sodium, sesquicarbonate de sodium, carbonate de potassium, bicarbonate de potassium, carbonate de lithium, bicarbonate de lithium, carbonate d'ammonium, bicarbonate d'ammonium, sesquicarbonate d'ammonium, et les mélanges de ceux-ci.

19. Un procédé de préparation d'une composition effervescente d'un agent thérapeutique à dissolution rapide, qui comprend:

(1) la formation d'une composition granulaire par dissolution d'un agent de granulation dans un solvant pour former une solution contenant de 1 à 50% en poids de l'agent de granulation, et mélange de l'agent thérapeutique avec la solution;

(2) le séchage de la composition granulaire;

(3) le calibrage de la composition granulaire séchée par broyage et tamisage pour obtenir des particules ayant une dimension comprise entre 100 et 400 μm;

(4) l'addition et le mélange d'un composé d'un premier système effervescent ayant une dimension particulaire comprise entre 50 et 600 μm avec la composition granulaire calibrée de l'étape (3) pour former un prémélange;

(5) le mélange d'un second système effervescent (qui peut être identique ou différent du premier système effervescent) avec le prémélange de l'étape (4) pour obtenir un mélange homogène de granules; et

(6) la récupération du produit.

20. Un procédé selon la revendication 19, dans lequel le composé du premier système effervescent de l'étape (4) est un acide.

21. Un procédé selon la revendication 19 ou 20, dans lequel l'agent thérapeutique est choisi parmi: acétaminophène, aspirine, ibuprofène et les mélanges de ceux-ci, l'agent thérapeutique étant de préférence l'acétaminophène ayant une masse volumique comprise entre 0,2 et 0,6 g/ml.

22. Un procédé selon l'une quelconque des revendications 19 à 21, comprenant de plus l'étape de conditionnement du mélange de l'étape (5) en comprimés.

**Revendications pour l'Etat contractant suivant : AT**

1. Une procédé de préparation d'une composition thérapeutique effervescente à dissolution rapide qui comprend:

(1) un prémélange de:

(a) un agent thérapeutique sous forme de granules ayant une dimension particulaire comprise entre 100 et 600 μm; et

(b) un composé d'un premier système effervescent, ce composé ayant une dimension particulaire comprise entre 50 et 600 μm; et

(2) un second système effervescent, qui peut être identique ou différent du premier système effervescent.

2. Un procédé selon la revendication 1, dans laquelle le prémélange est présent dans une proportion de 7 à 57,5% en poids de la composition finale et le système effervescent est présent dans une proportion de 42,5 à 90% en poids de la composition effervescente.

3. Un procédé selon la revendication 1 ou 2, dans lequel l'agent thérapeutique sous forme de granules comprend un agent thérapeutique et un agent de granulation.

4. Un procédé selon la revendication 3, dans laquelle l'agent de granulation est caractérisé en ce que:
   (a) il est soluble dans l'eau;
   (b) il a une viscosité inférieure à 100 cps (0.1 Pa·s), à 10% en poids dans de l'eau à 25°C; et
   (c) il est compatible avec l'agent thérapeutique.

5. Un procédé selon la revendication 3 ou 4, dans lequel l'agent de granulation est présent dans une proportion de 0,03 à 0,25% en poids de la composition effervescente.

6. Un procédé selon l'une quelconque des revendications 3 à 5, dans laquelle l'agent de granulation est choisi parmi: eau, alcool, polyvinylpyrrolidone, saccharose, hydroxypropylcellulose et les mélanges de ceux-ci, l'agent de granulation étant la polyvinylpyrrolidone.

7. Un procédé selon l'une quelconque des revendications 3 à 6, dans lequel l'agent thérapeutique est présent dans une proportion de 2 à 27% en poids de la composition effervescente.

8. Un procédé selon l'une quelconque des revendications 3 à 7, dans lequel l'agent thérapeutique est un analgésique choisi parmi: aspirine, acétaminophène, ibuprofène et les mélanges de ceux-ci, l'analgésique étant de préférence l'acétoaminophène ayant une masse volumique comprise entre 0,2 et 0,6 g/ml et, de préférence, ayant une dimension particulaire comprise entre 100 et 400 µm.

9. Un procédé selon l'une quelconque des revendications précédentes, dans lequel le composé du premier système effervescent mélangé avec l'agent thérapeutique sous forme de granules est présent dans une proportion de 5 à 30% en poids de la composition effervescente.

10. Un procédé selon l'une quelconque des revendications précédentes, dans lequel le second système effervescent est caractérisé par le dégagement d'un gaz non toxique quand il est mis en contact avec l'eau et par le fait qu'il est compatible avec l'agent thérapeutique.

11. Un procédé selon l'une quelconque des revendications précédentes, dans laquelle le second système effervescent comprend:
   un composé contenant du carbonate de préférence choisi parmi: carbonate de sodium, bicarbonate de sodium, sesquicarbonate de sodium, carbonate de potassium, bicarbonate de potassium, carbonate de lithium, bicarbonate de lithium, carbonate d'ammonium, bicarbonate d'ammonium, sesquicarbonate d'ammonium et les mélanges de ceux-ci; et
   un acide, de préférence choisi parmi: acide citrique, acide fumarique, acide adipique, acide malique, acide tartrique et les mélanges de ceux-ci, l'acide préféré étant l'acide citrique.

12. Un procédé selon la revendication 11, dans laquelle le composé contenant du carbonate est présent dans une proportion de 40 à 60% en poids de la composition effervescente.

13. Un procédé selon l'une quelconque des revendications précédentes, dans lequel le composé du premier système effervescent dans le prémélange est un acide ayant une dimension particulaire comprise entre 50 et 600 µm, de préférence choisi parmi: acide citrique, acide fumarique, acide adipique, acide malique, acide tartrique et les mélanges de ceux-ci, l'acide préféré étant l'acide citrique.

14. Un procédé selon la revendication 13 en combinaison avec la revendication 12, dans lequel le premier et le second systèmes effervescents sont identiques, et dans laquelle ledit acide particulaire du premier système effervescent est présent dans une proportion de 5 à 30% en poids de la composition effervescente de telle sorte que la teneur totale en acide de la composition effervescente soit comprise entre 22 et 46% en poids.

15. Un procédé de préparation d'une composition thérapeutique effervescente à dissolution rapide qui comprend:
   (1) un prémélange, présent dans une proportion de 7 à 57,5% de:

21

(a) un agent thérapeutique sous forme de granules ayant une dimension particulaire comprise entre 100 et 600 μm comprenant:

    (i) un agent thérapeutique présent dans une proportion de 2 à 27%; et

    (ii) un agent de granulation présent dans une proportion de 0,03 à 2,5%; et

(b) un acide ayant une dimension particulaire comprise entre 50 et 600 μm et étant présent dans une proportion de 5 à 30%; et

(2) un système effervescent présent dans une proportion de 42,5 à 90% et comprenant:

    (c) un composé contenant du carbonate dans une proportion de 40 à 60%; et

    (d) un acide dans une proportion de 2,5 à 30%, dans laquelle la teneur totale en acide des parties (b) et (d) précitées est comprise entre 22 et 46%, tous les pourcentages étant exprimés en poids par rapport au poids de la composition effervescente.

16. Un procédé selon la revendication 15, ayant une quelconque ou plusieurs caractéristiques des revendications 4, 6 et 8.

17. Un procédé selon la revendication 15 ou 16, dans lequel l'acide des parties (b) et (d) est choisi parmi: acide citrique, acide fumarique, acide adipique, acide malique, acide tartrique et les mélanges de ceux-ci, l'acide préféré étant l'acide citrique.

18. Un procédé selon la revendication 15, 16 ou 17, dans lequel le composé contenant du carbonate est choisi parmi: carbonate de sodium, bicarbonate de sodium, sesquicarbonate de sodium, carbonate de potassium, bicarbonate de potassium, carbonate de lithium, bicarbonate de lithium, carbonate d'ammonium, bicarbonate d'ammonium, sesquicarbonate d'ammonium, et les mélanges de ceux-ci.

19. Un procédé de préparation d'une composition effervescente d'un agent thérapeutique à dissolution rapide, qui comprend:

(1) la formation d'une composition granulaire par dissolution d'un agent de granulation dans un solvant pour former une solution contenant de 1 à 50% en poids de l'agent de granulation et mélange de l'agent thérapeutique avec la solution;

(2) le séchage de la composition granulaire;

(3) le calibrage de la composition granulaire séchée par broyage et tamisage pour obtenir des particules ayant une dimension comprise entre 100 et 600 μù;

(4) l'addition et le mélange d'un composé d'un premier système effervescent ayant une dimension particulaire comprise entre 50 et 600 μm avec la composition granulaire calibrée de l'étape (3) pour former un prémélange;

(5) le mélange d'un second système effervescent (qui peut être identique ou différent du premier système effervescent) avec le prémélange de l'étape (4) pour obtenir un mélange homogène de granules; et

(6) la récupération du produit.

20. Un procédé selon la revendication 19, dans lequel le composé du premier système effervescent de l'étape (4) est un acide.

21. Un procédé selon la revendication 19 ou 20, dans lequel l'agent thérapeutique est choisi parmi: acétaminophène, aspirine, ibuprofène et les mélanges de ceux-ci, l'agent thérapeutique étant de préférence l'acétaminophène ayant une masse volumique comprise entre 0,2 et 0,6 g/ml.

22. Une composition selon l'une quelconque des revendications 19 à 21, comprenant de plus l'étape de conditionnement du mélange de l'étape (5) en comprimés.